# EUROPÄISCHE PATENTANMELDUNG

(11) **EP 1 860 465 A2**
(43) Veröffentlichungstag der Anmeldung: **28.11.2007**
(21) Anmeldenummer: 07107345.6
(22) Anmeldetag: 02.05.2007
(51) Int. Cl.: G01T 1/29

(54) **Positronen-Emissions-Tomograph und Verfahren zur Ermittlung einer bei einer Strahlentherapie applizierten Dosisverteilung**

(30) Priorität: 23.05.2006 DE 102006024244
(71) Anmelder: SIEMENS AKTIENGESELLSCHAFT, 80333 München (DE)
(72) Erfinder: Braess, Henning, 91080, Uttenreuth (DE); Sommer, Andres, 91094, Langensendelbach (DE)

(57) **Zusammenfassung**

Um die Flexibilität und dadurch die Sensibilität eines Positronen-Emissions-Tomographen (2) zur Ermittlung einer bei einer Strahlentherapie applizierten Dosisverteilung zu verbessern, sind zwei um eine Drehachse (D) angeordnete Detektoren (10, 12) vorgesehen, die sich in radialer Richtung (16) gegenüberliegen und relativ zueinander beweglich gelagert sind.

## Beschreibung

Die Erfindung betrifft einen Positronen-Emissions-Tomograph sowie ein Verfahren zur Ermittlung einer bei einer Strahlentherapie applizierten Dosisverteilung.

Die Positronen-Emissions-Tomographie (PET) ist ein bildgebendes Verfahren der Nuklearmedizin, bei dem einem Patienten durch Injektion oder Inhalation ein radioaktiv markiertes Radiofarmakon, auch Tracer genannt, verabreicht wird. Beim Zerfall dieses Tracers werden Positronen ausgesendet. Ein Positron tritt nach kurzer Distanz in Wechselwirkung mit einem Elektron, wobei beide Teilchen vernichtet werden. An deren Stelle entsteht ein Paar von Gammaquanten, die sich in einem Winkel von 180° voneinander entfernen. Diese Gammaquanten durchdringen den Körper des Patienten und werden nach ihrem Austritt von zwei gegenüberliegenden Detektoren registriert.

Ein Positronen-Emissions-Tomographen zur Bildgebung umfasst üblicherweise mehrere Gammastrahlung-Detektoren, die nach Art eines Ringes den zu untersuchenden Patienten umschließen. Eine solche Anordnung ist beispielsweise in der DE 32 08 178 A1 beschrieben.

Bei einer Strahlentherapie, insbesondere einer Partikeltherapie, werden häufig Messungen vorgenommen, um zu prüfen, ob die geplante Strahlendosis mit der applizierten Dosis übereinstimmt. Als besonders geeignet für eine solche Dosisvalidierung bzw. Überwachung der Partikeltherapie haben sich PET-Systeme erwiesen. Allerdings sind ringförmige PET-Systeme für die Kombination mit einer Strahlentherapievorrichtung nicht geeignet, da sie den Strahlengang stören und die Positionierfreiheit des Patienten beschränken würden.

Es werden daher so genannte In-Beam PET-Systeme eingesetzt, die lediglich aus zwei gegenüberliegenden Detektoren bestehen. Die Öffnung zwischen den beiden Detektoren eines In-Beam PET ist derart dimensioniert, dass der Therapiestrahl zum Patienten gelangt, ohne auf die Detektoren zu treffen. Bei den bekannten In-Beam PET ist der Abstand zwischen den Detektoren fest und die Detektoren können entlang einer Drehachse der Gantry (in Einstrahlrichtung) verschoben werden. Da nur ein kleiner Teil der emittierten Strahlung durch die zwei Detektoren erfasst wird, sind die Detektoren außerdem um die Drehachse rotierbar gelagert.

Bei der Auslegung eines solchen In-Beam PET sind die Parameter, die sich auf seine Sensitivität auswirken, zu beachten. Die Sensitivität hängt von der Wahrscheinlichkeit ab, dass die Gammaquanten, die von einem Positron abgestrahlt werden, beide auf die Detektoren treffen. Wenn die Detektoren dicht beieinander liegen, deckt der In-Beam PET einen großen Raumwinkel ab, was zu einer großen Sensitivität führt und viel Information über die Strahlendosis gewonnen wird. Ein kleiner Detektorabstand ist außerdem aus Gründen der Bildqualität vorteilhaft. Gleichzeitig muss berücksichtigt werden, dass der Therapiestrahl Neutronen erzeugt, die nicht auf die Detektoren gelangen sollen. Die Neutronen werden in einem s.g. Neutronenkegel mit ca. 60° Öffnungswinkel in Strahlrichtung ausgesandt. Daher ist ein ausreichender Abstand zwischen den Detektoren für den Neutronenkegel erforderlich. Somit muss bei der Auslegung der herkömmlichen In-Beam PET ein Kompromiss in Bezug auf die Detektorsensitivität gefunden werden, der nicht immer zu den besten erreichbaren Ergebnissen führt.

Der Erfindung liegt die Aufgabe zugrunde eine Verbesserung der Sensitivität eines In-Beam PET zu ermöglichen.

Die Aufgabe wird erfindungsgemäß gelöst durch einen Positronen-Emissions-Tomograph zur Ermittlung einer bei einer Strahlentherapie applizierten Dosisverteilung, mit zwei um eine Drehachse angeordneten Detektoren, die sich in radialer Richtung gegenüberliegen, wobei die Detektoren relativ zueinander beweglich gelagert sind.

Die Erfindung basiert auf der Überlegung, dass die Sensitivität des In-Beam PET durch eine Erhöhnung seiner Positionierflexibilität erreicht wird. Hierbei kann der In-Beam PET auf der Gantry einer Strahlentherapievorrichtung montiert sein oder alternativ können die Detektoren auf einem Roboterarm befestigt sein und mit Hilfe des Roboterarms im Bereich der Strahlentherapievorrichtung um den Patienten gebracht werden. Die relative Bewegung der Detektoren zueinander gewährleistet dem Bedienpersonal des PET-Systems mehr Freiheit, um die Detektoren derart einzurichten, dass eine möglichst hohe Detektorsensitivität erreicht ist. Ein besonderer Vorteil hierbei ist, dass bei der Konfiguration der Detektoren auch patientenspezifische Parameter, wie z.B. die Größe des Patienten, berücksichtigt werden können.

Gemäß einer bevorzugten Ausgestaltung sind die Detektoren für voneinander entkoppelte Bewegungen über je ein Verstelllager gelagert. Durch die entkoppelte Bewegung der Detektoren ergeben sich deutlich mehr Möglichkeit, die Detektoren optimal zu positionieren und zu orientieren, um möglichst viele Informationen über die Dosisverteilung zu sammeln.

Vorzugsweise sind die Verstelllager für eine Verstellung der Detektoren in radialer Richtung ausgebildet. Hierbei erfolgt eine Höhenverstellung der Detektoren, die es ermöglicht, sowohl bei dünnen als auch bei dicken Patienten die Detektoren dicht an den Patienten zu fahren und somit eine besonders hohe Detektorsensitivität zu erreichen.

Gemäß einer weiteren bevorzugten Ausgestaltung sind Drehlager für eine Schwenkbewegung der Detektoren vorgesehen. Durch deren Schwenkbewegung werden die Detektoren außerhalb des Neutronenkegels positioniert, ohne dass sie weit vom Patienten wegbewegt werden müssen.

Die Detektoren sind bevorzugt um eine zur Drehachse parallele Schwenkachse schwenkbar angeordnet. Diese Ausführung ist konstruktiv besonders einfach, da die Kopplung der Detektoren an die Gantry ohnehin über diese Achse erfolgt. Somit sind zur Realisierung der Schwenkbewegung nur wenige technische Mittel erforderlich.

Gemäß einer bevorzugten Ausgestaltung sind die beiden Detektoren an einem Drehring angeordnet, so dass die beiden einzigen Detektoren in beliebigen Drehwinkeln und zueinander gegenüberliegende positioniert werden können.

Die Aufgabe wird ferner erfindungsgemäß gelöst durch ein Verfahren zur Ermittlung einer bei einer Strahlentherapie applizierten Dosisverteilung, mit zwei in radialer Richtung gegenüberliegenden Detektoren, die relativ zueinander bewegt werden. Die im Hinblick auf den Positronen-Emissions-Tomographen aufgeführten Vorteile und bevorzugten Ausführungsformen lassen sich sinngemäß auf das Verfahren übertragen.

Ein Ausführungsbeispiel der Erfindung wird im Folgenden anhand einer Zeichnung näher erläutert. Hierin zeigen in vereinfachter Darstellung:
- FIG 1: eine Vorderansicht In-Beam PET,
- FIG 2: eine Seitenansicht des In-Beam PET gemäß FIG 1,
- FIG 3: schematisch eine Höhenverstellung zweier Detektoren, und
- FIG 4: schematisch eine Schwenkbewegung zweier Detektoren.

Gleiche Bezugszeichen haben in den Figuren die gleiche Bedeutung.

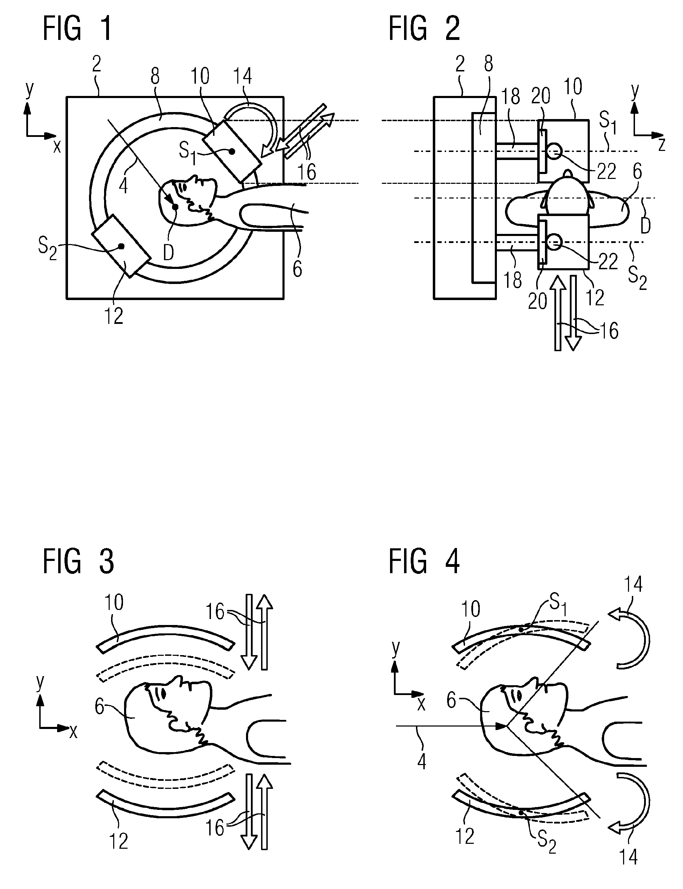

In FIG 1 ist schematisch ein In-Beam PET 2 gezeigt, der bei der Durchführung einer Partikeltherapie zur Ermittlung der bei der Therapie applizierten Dosisverteilung herangezogen wird. Bei der Partikeltherapie wird ein erkranktes Gewebe eines Patienten 6 mit einem von einem hier nicht dargestellten Teilchenbeschleuniger erzeugten Partikelstrahl 4 bestrahlt.

Um die Dosisverteilung des Partikelstrahls 4 zu überprüfen, sind auf einem um eine Drehachse D (hier mit einem Punkt angedeutet) rotierbaren Ring 8 zwei gegenüberliegende Detektoren 10, 12 angeordnet. Die Detektoren 10, 12 sind nach Art von Blöcken ausgebildet, die aus mehreren Szintillator-Kristallen (z.B. 32X32 Kristalle pro Detektor) mit nachgeschalteten Verstärkern bestehen. Da der Patient 6 während der Bestrahlung nicht bewegt werden kann, sind die Detektoren 10, 12 derart dimensioniert, dass sie einen möglicht großen Teil der von dem Partikelstrahl 4 erzeugten Gammaquanten registrieren, ohne dabei die Positionierfreiheit des Patienten 6 oder die Erzeugung und Ausrichtung des Partikelstrahls 4 störend zu beeinflussen. Die Detektoren 10, 12 sind weiterhin derart angetrieben, dass deren Bewegungen entkoppelt voneinander erfolgen und die Detektoren 10, 12 - bei fester 180°-Zuordnung - relativ zueinander verfahren werden können. Dadurch ist eine besonders günstige Positionierung der Detektoren 10, 12 bezüglich des Patienten 6 erzielt.

Die Detektoren 10, 12 sind beweglich gelagert, damit sie in eine für die Strahlendetektierung unter Berücksichtigung der Patientenbesonderheiten optimale Position gebracht werden können. Zunächst können sie gemeinsam entlang des Ringes 8 rotieren und horizontal entlang der Drehachse D verfahren werden (in FIG 1 senkrecht zur Papierebene). Darüber hinaus sind in diesem Ausführungsbeispiel die Detektoren 10, 12 um je eine Schwenkachse S₁, S₂, die parallel zur Drehachse D verläuft, drehbar lagert, was durch den Pfeil 14 gezeigt ist. Außerdem ist eine Höhenverstellung der Detektoren 10, 12 in einer radialen Richtung möglich, veranschaulicht durch die Pfeile 16, so dass die Detektoren 10, 12 dicht an den Patienten 6 verfahren werden.

Die Lagerung der beiden Detektoren 10, 12 an der Gantry 2 ist in FIG 2 dargestellt. Jeder der Detektoren 10, 12 ist von einer vom Ring 8 herausragenden Halterung 18 getragen. Die Detektoren 10, 12 weisen je ein Verstelllager 20 auf, über das sie in der radialen Richtung 16 verstellt werden. Diese Verstelllager 20 sind jeweils insbesondere nach Art einer Linearführung ausgebildet und beispielsweise von einem hier nicht dargestellten Elektromotor angetrieben. Ein weiteres Lagerelement, ein Drehlager 22 ist für die Schwenkbewegungen der Detektoren 10, 12 um die Schwenkachsen S₁, S₂ vorgesehen. Die Drehlager 22 sind in der FIG 2 durch die Kugeln 22 symbolisiert.

Wie die Höhenverstellung und die Schwenkbewegung der Detektoren 10, 12 erfolgt, wird nochmals in den Figuren 3 und 4 veranschaulicht. Durch die lineare Verstellbarkeit in Richtung 16 können die Detektoren 10, 12 dicht an den Patienten 6 herangefahren werden, was durch die gestrichelte Konturen der Detektoren 10, 12 angedeutet ist. Somit decken sie einen noch größeren Raumwinkel ab, was eine höhere Detektorsensitivität nach sich zieht. Hierbei wird insbesondere die Größe des Patienten 6 berücksichtigt. Bei kleinen und dünnen Patienten 6 können die Detektoren 10, 12 besonders nahe aneinander positioniert werden. Auch bei größeren und dickeren Patienten 6 werden die Detektoren 10, 12 patientenspezifisch optimal eingestellt. Die entkoppelte Bewegung beider Detektoren 10, 12 ermöglicht außerdem, dass sie unterschiedliche Abstände zum Patienten aufweisen, was ebenfalls aus der FIG 3 ersichtlich ist.

Durch die Öffnung zwischen den Detektoren 10, 12 kann der Partikelstrahl 4 zum Patienten 6 störungsfrei gelangen. Dieser Partikelstrahl 4 erzeugt im Körper des Patienten 6 Neutronen, die in einem s.g. Neutronenkegel mit ca. 60° Öffnungswinkel emittiert werden, wie aus FIG 4 zu entnehmen ist. Bei der Auslegung des In-Beam PET muss berücksichtigt werden, dass die entstandenen Neutronen auf die Detektoren 10, 12 nicht treffen dürfen. Dieses Problem wird in geschickter Weise durch die Schwenkbewegung der Detektoren 10, 12 behoben, was durch gestrichelte Linien gezeigt ist. Die entkoppelte Bewegung beider Detektoren 10, 12 ermöglicht, dass ein Detektor 10 um einen positivern Winkel und der gegenüberliege Detektor 12 um den gleichen negativen Winkel um die Schwenkachsen S₁, S₂ gedreht wird. Der besondere Vorteil hierbei ist, das es nicht notwendig ist, die Detektoren 10, 12 weit vom Patienten 6 zu verfahren.

## Patentansprüche

1. Positronen-Emissions-Tomograph (2) zur Ermittlung einer bei einer Strahlentherapie applizierten Dosisverteilung, mit zwei um eine Drehachse (D) angeordneten Detektoren (10, 12), die sich in radialer Richtung (16) gegenüberliegen, wobei die Detektoren (10, 12) relativ zueinander beweglich gelagert sind.

2. Positronen-Emissions-Tomograph (2) nach Anspruch 1, wobei die Detektoren (10, 12) für voneinander entkoppelte Bewegungen über je ein Verstelllager (20) gelagert sind.

3. Positronen-Emissions-Tomograph (2) nach Anspruch 2, wobei die Verstelllager (20) für eine Verstellung der Detektoren (10, 12) in radialer Richtung (16) ausgebildet sind.

4. Positronen-Emissions-Tomograph (2) nach einem der vorhergehenden Ansprüche, wobei Drehlager (22) für eine Schwenkbewegung der Detektoren (10, 12) vorgesehen sind.

5. Positronen-Emissions-Tomograph (2) nach Anspruch 4, wobei die Detektoren (10, 12) um eine zur Drehachse (D) parallele Schwenkachse (S₁, S₂) schwenkbar angeordnet sind.

6. Positronen-Emissions-Tomograph (2) nach einem der vorhergehenden Ansprüche, wobei die Detektoren (10, 12) auf einem um die Drehachse (D) rotierbaren Ring (8) angeordnet sind.

7. Verfahren zur Ermittlung einer bei einer Strahlentherapie applizierten Dosisverteilung, mit zwei in radialer Richtung (16) gegenüberliegenden Detektoren (10, 12), die relativ zueinander bewegt werden.
